# EUROPEAN PATENT SPECIFICATION

(11) **EP 0 568 622 B1**
(45) Date of publication and mention of the grant of the patent: **05.04.2000**
(21) Application number: 92904821.3
(22) Date of filing: 13.01.1992
(51) Int. Cl.: C12N 15/12, C07K 14/00, C12P 21/08

(54) **CORTICOTROPIN RELEASING FACTOR BINDING PROTEIN (CRF-bp)**
CORTICOTROPINAUSLÖSEFAKTOR-BINDENDES PROTEIN
PROTEINE DE LIAISON DU FACTEUR DE LIBERATION DE LA CORTICOTROPINE (CRF-bp)

(30) Priority: 15.01.1991 US 641341
(43) Date of publication of application: 10.11.1993
(73) Proprietor: THE SALK INSTITUTE FOR BIOLOGICAL STUDIES, La Jolla California 92037 (US); THE UNIVERSITY OF READING, Reading RG6 2AH (GB)
(72) Inventor: POTTER, Ellen, La Jolla, CA 92037 (US); BEHAN, Dominic, P., San Diego, CA 92131 (US); FISCHER, Wolfgang, H., Solana Beach, CA 92075 (US); LINTON, Elizabeth, A., South Oxon OX10 7LJ (GB); LOWRY, Philip, J., Reading Berks RG6 2HP (GB); VALE, Wylie, Walker, Jr., La Jolla, CA 92037 (US)
(74) Representative: Lawrence, Malcolm Graham
(86) International application number: US9200331
(87) International publication number: WO9213074

(56) References cited:
- Nature, vol. 349, 31 January 1991, E. POTTER et al.: "Cloning and characterization of the cDNAs for human and rat corticotropin releasing factor-binding proteins", pages 423-426, see the whole document
- J. Endocrinol., vol. 122, no. 1, July 1989, D.P. BEHAN et al.: "Isolation of the human plasma corticotrophin-releasing factor-binding protein", pages 23-31, see the whole document
- Biochem. Biophys. Res. Commun., vol. 165, no. 2, 15 December 1989, T. SUDA et al.: "Corticotropin-releasing factor-binding protein is a glycoprotein", pages 703-707, see the whole document

## Description

This invention relates to controlling the biological effect of CRF in mammals and more particularly to CRF binding proteins which can be employed to complex with CRF and thereby modulate CRF actions in mammals, as well as to antibodies to such binding proteins.

Corticotropin Releasing Factor, CRF, is a very potent stimulator of the synthesis and secretion of various peptides in the human body. Although CRF levels in human peripheral circulation are normally low, there are often elevated levels of CRF in the maternal circulation, which levels progressively increase throughout pregnancy. It is believed that this maternal plasma CRF most likely originates from the placenta wherein it plays a paracrine role. Placenta cells have been shown to respond to CRF and to produce CRF and its mRNA. Even though CRF concentrations measured in late gestational maternal plasma are similar to levels reported in rat hypothalamic portal blood, which levels are capable of stimulating ACTH release in vitro, it does not appear that there is normally overproduction of ACTH during pregnancy. However, maternal plasma ACTH concentrations do increase slightly with advancing gestation.

There have been reports of proteins (CRF-BP's) in human plasma which are capable of biologically inactivating CRF, such as Linton, E.A., et at, Clin. Endo. 28, 315-324 (1988) and Behan, D. P, et al. J. Endo. 122, 23-31 (1989) in the latter of which a partial purification process is disclosed wherein the purity of the isolated protein is estimated to be substantially higher than was later determined. It has been proposed that the role of this protein substance is the prevention of inappropriate pituitary-adrenal stimulation during pregnancy.

The human CRF-BP (CRF-binding protein) was purified to homogeneity and then the protein (hCRF-BP) partially characterized by amino acid sequence analysis. Thereafter, oligo primers were made and the cDNA corresponding to the hCRF-BP isolated and cloned, and the DNA sequence fully characterized, from human liver. Rat CRF-BP (rCRF-BP) cDNA was cloned from rat brain. The recombinant molecules have been expressed in COS cells and found to bind CRF with high affinity. The recombinant proteins can inhibit CRF binding to CRF antibodies and can inhibit CRF-induced ACTH release in vitro by pituitary cells.

These rat and human proteins bind to the 41-residue peptide which constitutes rat/human CRF (r/h CRF), the rat and human species having the same CRF molecule, the structure of which is set forth in U.S. Patent No. 4,489,163. As a result, these rat and human CRF-BPs can be administered to reduce high ACTH levels in mammals caused by excess CRF and can be used to treat Cushing's Disease and the like. These CRF-BPs are also useful in combating pituitary tumors that produce CRF. Moreover, they can be used to reduce pituitary ACTH secretion and hence reduce cortisol levels under any condition in which they are abnormally high, such as during chronic stress or in patients afflicted with anorexia nervosa or alcoholism. It has been found that CRF-BPs when administered intraveneously (IV) have also proved effective to prevent CRF-induced ACTH release. Furthermore, it is considered that IV administration of the CRF-BPs can be used to raise blood pressure and in this manner combat hypotension. The recombinant production of the CRF-BPs makes feasible their use in the foregoing manners. Antibodies have been made to these proteins, and these antibodies are useful for diagnostic assays to determine levels of the CRF-BPs and can also be used to purify the protein. Moreover, these antibodies are considered useful to counteract the biological effect of CRF-BPs in vivo.

By cloning the gene encoding human CRF-BP, recombinant expression of this protein is made feasible, and, as a result, methods of treatment can be carried out by the peripheral administration of the recombinant protein. Cloning of the human DNA was accomplished by the utilization of amino acid sequence data obtained by isolating the 37 kD protein from human plasma in purified form using micropreparative SDS polyacrylamide gel electrophoresis (PAGE). This separation produced a major band of the expected size of about 37 kD and a number of bands of impurities of higher and lower molecular weights. After transferring the separated proteins to nitrocellulose filter material, the band was selected and cut therefrom. The purified protein bound thereto was trypsinized, and the resultant tryptic fragments were separated from one another using reverse phase HPLC. These fragments were then individually subjected to Edman degradation, and sequence information was obtained from 7 separate tryptic fragments which were obtained from the 37 kD human protein. Following complete characterization of the clone as encoding a 322-residue precursor protein, it was determined that these seven tryptic fragments, that were first sequenced, constituted residues 30-45, residues 47-55, residues 112-119, residues 123-135, residues 152-162, residues 163-175, and residues 294-299.

Very generally, as a first step in the cloning, oligonucleotides were designed based upon residues 30-45 and residues 152-162. DNA from an adult human liver cDNA library was used as a template for the polymerase chain reaction (PCR) using various combinations of these oligonucleotides as primers. A liver cDNA library was chosen based upon the general knowledge that a number of serum binding proteins are synthesized by the liver.

More specifically, as a part of the original analysis, the human protein was partially purified from plasma using an hCRF-Sepharose affinity column, followed by gel filtration. The final step of micropreparative SDS gel electrophoresis was then carried out under standard conditions for such a micropreparative purification, and the resultant bands were transferred to nitrocellulose. The major protein band, corresponding to the binding protein, was excised and then treated with trypsin in situ, as is generally well known in this art, see P.N.A.S. 84, 6970-6974 (1987). The different tryptic fragments were recovered from the supernatant and resolved by RP-HPLC; they were then subjected individually to Edman degradation to obtain the amino acid sequences thereof. In addition, N-terminus sequence analysis of the overall protein, following its purification by the SDS gel electrophoresis step, was carried out by binding the separated bands to polyvinylidene difluoride filter material, excising the appropriate band, and then directly sequencing the pure material.

Following the sequencing of the seven tryptic fragments, two sets of degenerate oligonucleotide primers were made corresponding to tryptic fragment 30-45 and to tryptic fragment 152-162. A first degenerate oligonucleotide primer was designed based upon residues 33-43 in the first tryptic fragment, 
GA(T/C)TA(T/C)GATCCNTT(T/C) (C/T)TN(C/T)TNTT(T/C) (T/A)
(C/G)NGCNAAC.
 A second degenerate oligo primer was designed based upon residues 154-161 of the other tryptic fragment that was selected, CA(A/G)AA(T/C)GTNGCNATGATNTT(C/T)TTC.

DNA from a human adult liver cDNA library was used as a template in 25 cycles of PCR, with 1 minute of denaturation at 94°C, 2 minutes of annealing at 45°C, and 3 minutes of extension at 72°C. The PCR products were analyzed on a 1% (w/v) TBE agarose gel; a 387 bp fragment was electroeluted into a 12 M ammonium acetate solution using an IBI model UEA Bio-Rad electroeluter. The 387 PCR fragment was subcloned into the Sma I site of Bluescript KS vector, and nucleotide sequencing was then carried out using the Sanger dideoxy chain termination method using Sequenase (USB). The sequenced DNA fragment contained an open reading frame which also encoded tryptic fragments 47-55, 112-119, and 123-135, along with peptides corresponding to the above two oligonucleotide primers on the 5' and 3' ends.

The coding region from this PCR subclone was random-primed and then used to screen the original human liver cDNA library.

Duplicate nitrocellulose filters were hybridized in 50% formamide, 5 parts SSC buffer, 1 part Denhardt's solution, 0.1% SDS, 100 µg/ml sheared salmon sperm DNA and ³²P labelled insert (1 x 10⁶ cpm/ml) 42°C for 18 hours. Filters were washed at 60°C in 2 x SSC. Two partial overlapping clones for the hCRF-BP coding region were isolated containing inserts 650 bp and 570 bp, respectively. The inserts were subcloned, sequenced and shown to contain partial cDNA sequences for the human CRF-BP.

A new library using adult human liver RNA was constructed in order to obtain full length cDNA clones. mRNA was isolated by guanidium isothiocynate-caesium chloride method and oligo dT chromotography. 10µg mRNA was used for the λ-Zap II cloning system (Stratagene) which made a library with a 5 x 10⁶ bases. 1 x 10⁶ plaques were screened with inserts from the two partial, overlapping clones. Seven clones were identified, one of which contained a 1.8 kb insert with an open reading frame coding for a 322 amino acid protein which contained all of the amino acid sequences from the tryptic fragments of the purified hCRF-BP and which has its deduced AA sequence set forth hereinafter as SEQ ID No: 1. As noted hereinafter, this amino acid sequence of 322 residues is believed to include a 24-residue signal sequence:-

Met Ser Pro Asn Phe Lys Leu Gln Cys His Phe Ile Leu Ile Phe Leu
-20 -15 -10

Thr Ala Leu Arg Gly Glu Ser Arg Tyr Leu Glu Leu Arg Glu Ala Ala
-5 1 5

Asp Tyr Asp Pro Phe Leu Leu Phe Ser Ala Asn Leu Lys Arg Asp Val
10 15 20

Ala Gly Glu Gln Pro Tyr Arg Arg Ala Leu Arg Cys Leu Asp Met Leu
25 30 35 40

Ser Leu Gln Gly Gln Phe Thr Phe Thr Ala Asp Arg Pro Gln Leu His
45 50 55

Cys Ala Ala Phe Phe Ile Ser Glu Pro Glu Glu Phe Ile Thr Ile His
60 65 70

Tyr Asp Gln Val Ser Ile Asp Cys Gln Gly Gly Asp Phe Leu Lys Val
75 80 85

Phe Asp Gly Trp Ile Leu Lys Gly Glu Lys Phe Pro Ser Ser Gln Asp
90 95 100

His Pro Leu Pro Ser Ala Glu Arg Tyr Ile Asp Phe Cys Glu Ser Gly
105 110 115 120

Leu Ser Arg Arg Ser Ile Arg Ser Ser Gln Asn Val Ala Met Ile Phe
                125                 130                 135

Phe Arg Val His Glu Pro Gly Asn Gly Phe Thr Leu Thr Ile Lys Thr
            140                 145                 150

Asp Pro Asn Leu Phe Pro Cys Asn Val Ile Ser Gln Thr Pro Asn Gly
        155                 160                 165

Lys Phe Thr Leu Val Val Pro His Gln His Arg Asn Cys Ser Phe Ser
    170                 175                 180

Ile Ile Tyr Pro Val Val Ile Lys Ile Ser Asp Leu Thr Leu Gly His
185                 190                 195                 200

Val Asn Gly Leu Gln Leu Lys Lys Ser Ser Ala Gly Cys Glu Gly Ile
                205                 210                 215

Gly Asp Phe Val Glu Leu Leu Glu Gly Thr Gly Leu Asp Pro Ser Lys
            220                 225                 230

Met Thr Pro Leu Ala Asp Leu Cys Tyr Pro Phe His Gly Pro Ala Gln
        235                 240                 245

Met Lys Val Gly Cys Asp Asn Thr Val Val Arg Met Val Ser Ser Gly
    250                 255                 260

Lys His Val Asn Arg Val Thr Phe Glu Tyr Arg Gln Leu Glu Pro Tyr
265                 270                 275                 280

Glu Leu Glu Asn Pro Asn Gly Asn Ser Ile Gly Glu Phe Cys Leu Ser
                285                 290                 295

Gly Leu

The nucleotide sequence of the cDNA clone is shown hereinafter as SEQ ID NO:2 (with the encoded amino acid sequence set forth immediately below each codon):

GGACCTCCGG AGCAGACAGC ACAGCAGCTG CAGAGGCAAG GCCAGC ATG TCG CCC 55
Met Ser Pro

AAC TTC AAA CTT CAG TGT CAC TTC ATT CTC ATC TTC CTG ACG GCT CTA 103
Asn Phe Lys Leu Gln Cys His Phe Ile Leu Ile Phe Leu Thr Ala Leu
-20 -15 -10

AGA GGG GAA AGC CGG TAC CTA GAG CTG AGG GAA GCG GCG GAC TAC GAT 151
Arg Gly Glu Ser Arg Tyr Leu Glu Leu Arg Glu Ala Ala Asp Tyr Asp
-5 1 5 10

CCT TTC CTG CTC TTC AGC GCC AAC CTG AAG CGG GAC GTG GCT GGG GAG 199
Pro Phe Leu Leu Phe Ser Ala Asn Leu Lys Arg Asp Val Ala Gly Glu
15 20 25

CAG CCG TAC CGC CGC GCT CTG CGG TGC CTG GAC ATG CTG AGC CTC CAG 247
Gln Pro Tyr Arg Arg Ala Leu Arg Cys Leu Asp Met Leu Ser Leu Gln
30 35 40

GGC CAG TTC ACC TTC ACC GCC GAC CGG CCG CAG CTG CAC TGC GCA GCC 295
Gly Gln Phe Thr Phe Thr Ala Asp Arg Pro Gln Leu His Cys Ala Ala
45 50 55

TTC TTC ATC AGC GAG CCC GAG GAG TTC ATT ACC ATC CAC TAC GAC CAG 343
Phe Phe Ile Ser Glu Pro Glu Glu Phe Ile Thr Ile His Tyr Asp Gln
60 65 70 75

GTC TCC ATC GAC TGT CAG GGC GGC GAC TTC CTG AAG GTA TTT GAT GGT 391
Val Ser Ile Asp Cys Gln Gly Gly Asp Phe Leu Lys Val Phe Asp Gly
80 85 90

TGG ATT CTC AAG GGG GAG AAG TTC CCC AGT TCC CAG GAT CAT CCT CTC 439
Trp Ile Leu Lys Gly Glu Lys Phe Pro Ser Ser Gln Asp His Pro Leu
95 100 105

CCC TCA GCT GAG CGG TAC ATA GAT TTC TGT GAG AGT GGT CTT AGC AGG 487
Pro Ser Ala Glu Arg Tyr Ile Asp Phe Cys Glu Ser Gly Leu Ser Arg
110 115 120

AGG AGC ATC AGA TCT TCC CAG AAT GTG GCC ATG ATC TTC TTC CGA GTC 535
Arg Ser Ile Arg Ser Ser Gln Asn Val Ala Met Ile Phe Phe Arg Val
125 130 135

CAT GAA CCA GGA AAT GGA TTC ACA TTA ACC ATA AAG ACA GAC CCC AAC 583
His Glu Pro Gly Asn Gly Phe Thr Leu Thr Ile Lys Thr Asp Pro Asn
140 145 150 155

CTC TTT CCT TGC AAT GTC ATT TCT CAG ACT CCA AAT GGA AAG TTT ACC 631
Leu Phe Pro Cys Asn Val Ile Ser Gln Thr Pro Asn Gly Lys Phe Thr
160 165 170

CTG GTA GTT CCA CAC CAG CAT CGA AAC TGC AGC TTC TCC ATA ATT TAT 679
Leu Val Val Pro His Gln His Arg Asn Cys Ser Phe Ser Ile Ile Tyr
175 180 185

CCT GTG GTG ATC AAA ATA TCT GAT CTT ACC CTG GGA CAC GTA AAT GGT 727
Pro Val Val Ile Lys Ile Ser Asp Leu Thr Leu Gly His Val Asn Gly
190 195 200

CTT CAG TTA AAG AAA TCC TCA GCA GGT TGC GAG GGA ATA GGA GAC TTT 775
Leu Gln Leu Lys Lys Ser Ser Ala Gly Cys Glu Gly Ile Gly Asp Phe
205 210 215

GTG GAG CTG CTG GAG GGA ACT GGA TTG GAC CCT TCC AAG ATG ACG CCT 823
Val Glu Leu Leu Glu Gly Thr Gly Leu Asp Pro Ser Lys Met Thr Pro
220 225 230 235

TTA GCT GAT CTC TGC TAC CCC TTT CAT GGC CCG GCC CAG ATG AAA GTT 871
Leu Ala Asp Leu Cys Tyr Pro Phe His Gly Pro Ala Gln Met Lys Val
240 245 250

GGC TGT GAC AAC ACT GTG GTG CGC ATG GTC TCC AGT GGA AAA CAC GTA 919
Gly Cys Asp Asn Thr Val Val Arg Met Val Ser Ser Gly Lys His Val
255 260 265

AAT CGT GTG ACT TTT GAG TAT CGT CAG CTG GAG CCG TAC GAG CTG GAA 967
Asn Arg Val Thr Phe Glu Tyr Arg Gln Leu Glu Pro Tyr Glu Leu Glu
270 275 280

AAC CCA AAT GGA AAC AGT ATC GGG GAA TTC TGT TTG TCT GGT CTT TGA 1015
Asn Pro Asn Gly Asn Ser Ile Gly Glu Phe Cys Leu Ser Gly Leu .
285 290 295

ATAACCAACC CAGTGATTTA CATGCTGATA GCTAAGTGAG TTTTTAATGG CCATTGTGTA 1075

TGATTTTGAT GCACAACTAG TTAAAAGCCT TTCATACCAG TCAGTATTTC CCAGCCTTGA 1135

GCGCACGCAC ACACCACACA CATACACACA CGCATTATTT TTGTTACTTT GCTTCTTTTT 1195

ATGTTTGTAA TCTGTAAATG AACACATGGC AGAAAATAAC CCTGATTGGT AGG 1248

Based upon N-terminal sequencing of the purified hCRF-BP, it is determined that the mature protein begins at residue 25 and that, as noted above, a N-terminal 24-residues constitutes a signal sequence, as represented hereinabove. A putative N-glycosylation site is found in the predicted sequence at residue 180 (based upon the 298-residue sequence), which is consistent with the presence of asparagine-linked sugar moieties in the native hCRF-BP. Analysis of the full length sequence for hydrophobicity, using the Kyte and Doolittle program, revealed a pattern of randomly dispersed hydrophobic and hydrophilic sections characteristic of a soluble protein. There are 10 interdispersed cysteine residues (excluding the 24-residue signal sequence) which suggests the potential presence of five intramolecular disulfide bonds. This is consistent with the experimental data that the reduced form of the purified hCRF-BP exhibits a higher apparent molecular weight, than does the non-reduced form, when run on an SDS gel--a characteristic of a protein containing disulfide bonds.

As previously indicated, the CRF protein of the human species has the exact same 41 amino acid sequence as the CRF protein of the rat species, from which fact homology between the critical regions of the binding proteins can be fairly predicted. mRNA from rat brain was screened for mRNA for CRF-BP, and the target mRNA was detected. Thereafter, a rat cortex cDNA library was screened, using as a hybridization probe the human cDNA corresponding to the purified hCRF-BP referred to earlier, and several clones were isolated. One clone contained a 1.85 kb insert which was sequenced; it predicted a 322 amino acid precursor protein that is 84% identical to human CRF-BP. The deduced amino acid sequence of the rat species is set forth hereinbelow as SEQ ID NO:3:

Met Ser Pro Asn Phe Lys Leu Gln Cys His Phe Thr Leu Ile Leu Leu
-20 -15 -10
Thr Ala Leu Arg Gly Glu Ser Arg Tyr Leu Glu Val Gln Glu Ala Ala
-5 1 5
Val Tyr Asp Pro Phe Leu Leu Phe Ser Ala Asn Leu Lys Arg Asn Leu
10 15 20
Ala Glu Glu Gln Pro Tyr Arg Arg Ala Leu Arg Cys Leu Asp Met Leu
25 30 35 40
Ser Leu Pro Gly Gln Phe Thr Phe Thr Ala Asp Gln Pro Gln Leu His
45 50 55
Cys Ala Ala Phe Phe Ile Gly Glu Pro Glu Glu Phe Ile Thr Ile His
60 65 70
Phe Asp Leu Val Ser Ile Asp Cys Gln Gly Gly Asp Phe Leu Lys Val
75 80 85
Phe Asp Gly Trp Ile Leu Lys Gly Glu Lys Phe Pro Ser Ser Gln Asp
90 95 100
His Pro Leu Pro Thr Arg Glu Arg Tyr Thr Asp Phe Cys Glu Ser Gly
105 110 115 120

Leu Thr Arg Arg Ser Val Thr Ser Ser Gln Asn Val Ala Met Val Phe
                125                 130                 135

Phe Arg Val His Glu Pro Gly Asn Gly Phe Thr Ile Thr Ile Lys Thr
            140                 145                 150

Asp Pro Asn Leu Phe Pro Cys Asn Ile Ile Ser Gln Thr Pro Ser Gly
        155                 160                 165

Arg Phe Ala Leu Val Val Pro Tyr Gln His Gln Asn Cys Ser Phe Ser
    170                 175                 180

Ile Ile Tyr Pro Val Thr Ile Lys Ile Ser Asp Leu Ala Leu Gly His
185                 190                 195                 200

Leu His Gly Leu Gln Leu Lys Lys Pro Ala Ala Gly Cys Gly Gly Thr
                205                 210                 215

Gly Asp Phe Val Glu Leu Leu Gly Gly Thr Gly Leu Asp Thr Ser Lys
            220                 225                 230

Met Met Leu Leu Val Asp Leu Cys Tyr Pro Phe His Gly Pro Ala Gln
        235                 240                 245

Met Lys Ile Ser Cys Asp Asn Ala Val Val Arg Met Val Ser Ser Gly
    250                 255                 260

Lys His Met Asn Arg Val Thr Phe Glu Tyr Arg Gln Leu Glu Pro Leu
265                 270                 275                 280

Glu Leu Glu Thr Ser Thr Arg Asn Ser Ile Pro Glu Tyr Cys Leu Ser
                285                 290                 295

Ser Leu

The nucleotide sequence of the clone from which the sequence was deduced is set forth hereinafter as SEQ ID NO:4, with the encoded amino acid sequence shown immediately below each codon:

AAAGAGACCC AGGAAAGGAC CCTAGCAGCT TCGAGTTCTC AGTGTGGGCG AAGGCGAGGG 60

AAGAAACGCC TAAGATCTCC GCAGCCGAGC TCACCAGCTG CAGACACAAG GCCAGCC 117

ATG TCA CCG AAC TTC AAA CTC CAA TGC CAC TTC ACT CTG ATC CTC CTG 165
Met Ser Pro Asn Phe Lys Leu Gln Cys His Phe Thr Leu Ile Leu Leu
-24 -20 -15 -10

ACA GCT CTA AGG GGA GAG AGC CGC TAC CTA GAG GTG CAA GAA GCC GCA 213
Thr Ala Leu Arg Gly Glu Ser Arg Tyr Leu Glu Val Gln Glu Ala Ala
-5 1 5

GTC TAC GAC CCT TTC CTG CTT TTC AGC GCC AAT TTG AAG CGG AAC CTG 261
Val Tyr Asp Pro Phe Leu Leu Phe Ser Ala Asn Leu Lys Arg Asn Leu
10 15 20

GCA GAG GAG CAG CCC TAC CGA CGG GCT CTG CGC TGC CTG GAC ATG CTG 309
Ala Glu Glu Gln Pro Tyr Arg Arg Ala Leu Arg Cys Leu Asp Met Leu
25 30 35 40

AGC CTC CCT GGC CAG TTC ACC TTC ACC GCT GAC CAG CCG CAG CTG CAC 357
Ser Leu Pro Gly Gln Phe Thr Phe Thr Ala Asp Gln Pro Gln Leu His
45 50 55

TGC GCC GCC TTC TTC ATC GGC GAG CCG GAG GAG TTC ATC ACC ATC CAC 405
Cys Ala Ala Phe Phe Ile Gly Glu Pro Glu Glu Phe Ile Thr Ile His
60 65 70

TTT GAC CTG GTC TCC ATC GAC TGC CAG GGT GGG GAT TTC CTG AAG GTA 453
Phe Asp Leu Val Ser Ile Asp Cys Gln Gly Gly Asp Phe Leu Lys Val
75 80 85

TTT GAT GGT TGG ATC CTT AAG GGG GAG AAG TTC CCA AGT TCT CAG GAT 501
Phe Asp Gly Trp Ile Leu Lys Gly Glu Lys Phe Pro Ser Ser Gln Asp
90 95 100

CAC CCT CTG CCC ACC AGG GAG AGG TAC ACA GAT TTC TGT GAG AGC GGT 549
His Pro Leu Pro Thr Arg Glu Arg Tyr Thr Asp Phe Cys Glu Ser Gly
105 110 115 120

CTC ACC AGA AGG AGT GTT ACA TCT TCC CAG AAT GTG GCC ATG GTC TTC 597
Leu Thr Arg Arg Ser Val Thr Ser Ser Gln Asn Val Ala Met Val Phe
125 130 135

TTC CGG GTC CAT GAA CCA GGA AAT GGA TTC ACG ATA ACC ATA AAG ACA 645
Phe Arg Val His Glu Pro Gly Asn Gly Phe Thr Ile Thr Ile Lys Thr
140 145 150

GAC CCC AAC CTC TTC CCT TGC AAT ATC ATC TCT CAG ACT CCG AGT GGA 693
Asp Pro Asn Leu Phe Pro Cys Asn Ile Ile Ser Gln Thr Pro Ser Gly
155 160 165

AGA TTT GCT TTG GTG GTT CCA TAC CAG CAC CAA AAC TGC AGC TTT TCC 741
Arg Phe Ala Leu Val Val Pro Tyr Gln His Gln Asn Cys Ser Phe Ser
170 175 180

ATC ATT TAT CCG GTG ACC ATC AAA ATC TCT GAC CTC GCC CTG GGA CAC 789
Ile Ile Tyr Pro Val Thr Ile Lys Ile Ser Asp Leu Ala Leu Gly His
185 190 195 200

CTG CAT GGC CTT CAG TTG AAG AAA CCT GCG GCT GGC TGT GGT GGA ACT 837
Leu His Gly Leu Gln Leu Lys Lys Pro Ala Ala Gly Cys Gly Gly Thr
205 210 215

GGA GAC TTT GTG GAG CTG CTG GGA GGA ACT GGA CTG GAC ACC TCC AAG 885
Gly Asp Phe Val Glu Leu Leu Gly Gly Thr Gly Leu Asp Thr Ser Lys
220 225 230

ATG ATG CTC TTA GTG GAC CTG TGT TAC CCC TTT CAT GGC CCT GCC CAG 933
Met Met Leu Leu Val Asp Leu Cys Tyr Pro Phe His Gly Pro Ala Gln
235 240 245

ATG AAA ATT AGC TGC GAC AAT GCT GTG GTG AGG ATG GTC TCC AGT GGA 981
Met Lys Ile Ser Cys Asp Asn Ala Val Val Arg Met Val Ser Ser Gly
250 255 260

AAA CAC ATG AAC CGT GTG ACT TTT GAG TAT CGT CAG CTG GAA CCA CTC 1029
Lys His Met Asn Arg Val Thr Phe Glu Tyr Arg Gln Leu Glu Pro Leu
265 270 275 280

GAG CTG GAA ACC TCG ACC AGA AAC AGC ATC CCG GAG TAC TGC TTG TCT 1077
Glu Leu Glu Thr Ser Thr Arg Asn Ser Ile Pro Glu Tyr Cys Leu Ser
285 290 295

AGT CTT TGA ATGACCAGC 1095
Ser Leu

The applicants define their invention as DNA encoding CRF-binding protein (CRF-BP), said DNA being DNA as defined in one of integers A, B, C and D below :-
A. recombinant DNA comprising a DNA sequence encoding the AA sequence of an human CRF (hCRF) binding protein (hCRF-BP) which binds to hCRF to render inactive said hCRF, said DNA sequence being hybridizable to the DNA defined in integer B below and being obtainable by the following specific procedures:-
   (i) purifying hCRF-BP from human plasma by a purification process comprised of:-
      (a) elution of the human plasma through an hCRF-Sepharose affinity column,
      (b) gel filtration of the eluting fraction,
      (c) micropreparative SDS gel electrophoresis,
      (d) transfer of the resulting bands to nitrocellulose, and
      (e) excising the major protein band;
   (ii) obtaining two tryptic fragments identified by the AA sequences set forth in integer (iii) below from said excised protein band by a process comprised of:-
      (a) trypsinising the excised protein band by treatment with typsin, and
      (b) recovering a fraction comprising the aforesaid two tryptic fragments from the supernatant formed in the trypsinising treatment, followed by RP-HPLC resolution thereof
   (iii) advancing to an olignucleotide primer preparation stage two polypeptides, respectively having the AA sequences set forth below, said polypeptides being the resolved tryptic fragments obtained from step (ii)(b) aforesaid:-
      Glu-Ala-Ala-Asp-Tyr-Asp-Pro-Phe-Leu-Leu-Phe-Ser-Ala-Asn-Leu-Lys
      and
      Ser-Ser-Gln-Asn-Val-Ala-Met-Ile-Phe-Phe-Arg
   (iv) forming the two sets of degenerate oligonucleotide primers defined below and corresponding to portions of the aforesaid tryptic fragment AA sequences:- 
(Asp) (Tyr) Asp Pro (Phe) (Leu) (Leu) (Phe)
GA (T/C) TA(T/C) GAT CCN TT (T/C) (C/T) TN (C/T) TN TT (T/C)

(Ser) (Ala) Asn
(T/A) (C/G) N GCN AAC; and

(Gln) (Asn) ValAlaMetIle(Phe) Phe
CA (A/G) AA (T/C) GTNGCNATGATNTT (C/T) TTC
   (v) performing multiple cycles of PCR using DNA from a human adult liver cDNA library as a template to obtain a DNA fragment encoding a peptide containing said two AA sequences, and
   (vi) using said DNA fragment as a probe to screen said human liver cDNA library to obtain DNA encoding human CRF-BP containing said two AA sequences;
B. recombinant DNA comprising a DNA sequence encoding the AA sequence of a rat CRF (rCRF) binding protein (rCRF-BP) which binds to hCRF to render inactive said hCRF, said rCRF-BP having AA SEQ ID NO: 3;
C. recombinant DNA comprising a DNA sequence encoding the AA sequence of a homolog of said hCRF-BR or of said rCRF-BP which homolog is native to another mammalian species and binds to hCRF to render inactive said hCRF,
D. recombinant DNA comprising a DNA sequence encoding the AA sequence of a CRF-binding AA fragment of hCRF-BP, rCRF-BP or hCRF-BP or rCRF-BP homolog as defined in integer C above.

Included within the scope of the invention is a recombinant protein having the following characteristics:-
(i) it complexes to corticotropin releasing factor ;
(ii) it contains an AA sequence as defined below:-
   (a) an AA sequence as encoded by hCRF-BP DNA as defined in integer A above;
   (b) an AA sequence of rCRF-BP as defined in integer B above, namely AA SEQ ID NO: 3;
   (c) an AA sequence of an hCRF-BP or rCRF-BP homolog as defined in integer C above; or
   (d) an AA sequence as defined in integer D above of a CRF-binding AA fragment
(iii) it has a purity of at least about 98% ; and
(iv) it binds to hCRF so as to effect biological inactivity thereof.

All ten of the cysteine residues shown in the AA sequences set forth above and the putative N-glycosylation site referred to appear at exactly the same residues in the rat sequence as in the human sequence; this conservation between the human and rat CRF-BPs suggests that these residues may play an important role in the structure/function of CRF-BP.

Because the full length rCRF-BP clone was isolated from an Okayama-Berg library, it thus contained an SV40 promoter and polyadenylation signal, thereby rendering it suitable for transfection into COS cells, see Mol. Cell. Biol. 2, 161-170 (1982). The full length cDNA insert of the hCRF-BP was subcloned into a similar vector pSG5 (Stratagene) which contains an SV40 promoter, β-globulin splice site and an SP40 polyadenylation signal. The expression constructs for both rat and human CRF-BP were transfected into COS cells, and the media from the cells was collected. Cells which were transfected with the cDNAs for either hCRF-BP or rCRF-BP secreted proteins which were effective to inhibit CRF binding to an anti-CRF antibody. More specifically, the 1.8 kb insert which includes the nucleotide sequence coding for the human protein was excised with XhoI (filled-in)-Bam HI and subcloned into the Bgl II (filled-in)-Bam site of pSG5 vector(Stratagen). COS7 cells were transiently transfected using DEAE-Dextran with SV40 expression vectors containing cDNA inserts for human and rat CRF-BP. Media was collected 72 hours after transfection. Various dilutions of conditioned media were incubated with ¹²⁵I-rCRF trace and a 1:6000 dilution of rabbit anti-CRF antibody for 30 minutes at room temperature. Samples were precipitated with sheep anti-rabbit gamma globulin and 10% polyethyleneglycol (PEG). Following washing with SPEA buffer (50 mM sodium phosphate, 100 mM sodium chloride, 25 mM EDTA, 0.1% sodium azide), the precipitates were centrifuged, and the pellets radioactively counted.

The CRF-BP proteins obtained from the media from the COS cells demonstrated bioactivity to inhibit CRF-induced ACTH release from primary rat pituitary cells in a competitive manner. The results of this experimental data show that the rat CRF-BP and the human CRF-BP are substantially equally effective in inhibiting CRF-induced ACTH release, which is not unexpected insofar as the two native CRF proteins have the exact amino acid sequence. In these experiments, the conditioned media was placed on primary and carrier pituitary cell cultures using known techniques, as earlier described in Vale, W., et al., Methods in Enzymology - Hormone Action:Neuropeptides (Academic Press) 124, 389-401 (1986). Varying concentrations of rCRF were added to the media, and the cultures were incubated for 3 hours. The media was then removed and assayed for ACTH by double antibody RIA (Diagnostic Products Corp.). As a result of these tests, it is considered that bioactivity of CRF is abolished as a result of binding between CRF and CRF-BPs, and thus, it is considered that the CRF-BPs can be administered so as to treat hypertension thought to be caused by elevated CRF levels as in the case of pregnancy-induced hypertension. IV administration of 50 µg of CRF-BP to male rats, followed in one minute by 5 µg of r/h CRF, showed no rise in plasma ACTH over 30 minutes, proving the effectiveness of CRF-BP administration in vivo.

Analyses of the human and rat CRF-BPs show that the recombinantly produced binding proteins have the same high affinity for human/rat CRF as that exhibited by the purified human CRF-BP (K_{d} = 0.1 ± 0.2 nM). However, the experimental data shows that the recombinant CRF-BPs bind ovine CRF with a much lower affinity. This indicates a difference between the binding protein and the pituitary CRF receptor which does not significantly distinguish between binding to h/rCRF and binding to oCRF. Moreover, it appears that the CRF-BPs have as high or higher affinity for h/rCRF than do CRF receptors. CRF and its target cell receptors are broadly distributed throughout the central nervous system and in a number of peripheral tissues, including placenta, adrenal, sympathetic ganglia, lymphocytes, gastrointestinal tract, pancreas and gonads. Generally, CRF is produced and acts in a trans-synaptic, paracine or neuroendocrine fashion. It appears that the plasma CRF-BP provides a mechanism to protect human beings from hormonally significant concentrations of CRF and thereby protects the integrity of this restricted system especially during pregnancy. The presence of mRNA for CRF-BP in the brains of primates and rats suggests that this protein co-localizes to some CRF pathways and modulates the neural roles of the neuropeptide CRF.

Antibodies to these CRF-BP proteins of either monoclonal or polyclonal form can be produced using techniques presently known in the art, and antibodies which are effective to counteract the effects of CRF-BP can be elicited using only the synthetic N-terminal segment of the human or rat protein. For example, antibodies raised in rabbits against a synthetic peptide, representing the amino terminal sequence of human CRF-BP, recognize the synthetic peptide and the CRF-BP on an equimolar basis, and they are capable of inhibiting the activity of the native protein in vitro. Amino terminal-directed antibodies to CRF-BP may be obtained by immunizing three month old male and female white New Zealand rabbits with the synthetic peptide to which Tyr has been added at the C-terminus in order to couple it, as an antigen, to BSA by a bisdiazotized benzidine(BDB) linkage by reaction for 2 hours at 4°C. The reaction mixture is dialized to remove low molecular weight material, and the retentate is frozen in liquid nitrogen and stored at -20°C. Animals are immunized with the equivalent of 1 mg of the peptide antigen according to the procedure of Benoit et al. P.N.A.S. USA, 79, 917-921 (1982). At four week intervals, the animals are boosted by injections of 200 µg of the antigen and bled ten to fourteen days later. After the third boost, antiserum is examined for its capacity to bind radioiodinated antigen peptide prepared by the chloramine-T method and then purified by CMC-ion exchange column chromatography.

A radioimmunoassay is established with the antisera and serum from subsequent bleeds from the same rabbits. The native protein is recognized by the antibodies on an equimolar basis as compared to the synthetic peptide antigen. These antibodies are considered to be capable of at least partially neutralizing the biological activity of the CRF-BP, and substantially all such activity can likely be neutralized when higher amounts of antibodies are used. It is believed that immunoaffinity or affinity chromatography can also be applied to achieve the purification of CRF-BP from serum or from other biological materials.

These antibodies can be used in assays for detecting the levels of CRF-BP in mammals, particularly humans. The antibodies can also be used for treatment to neutralize the effect of CRF-BP in mammals and should also prove to be quite useful for diagnostic test kits and the like.

As previously indicated, it is most likely that there is internal disulfide-bonding between cysteine residues of the chain. Mammalian CRF-BP polypeptides produced by recombinant DNA techniques are inherently biologically active, perhaps because the three-dimensional structure which the CRF-BP assumes within cells is the structure which is adapted to binding to the native CRF protein. The three-dimensional structure which the molecule assumes through natural folding and through hydrophobic and hydrophilic interactions with aqueous media may promote desired bonding or non-bonding between cysteine residues. Also, enzymatic regulatory mechanisms within cells may help to ensure desired disulfide bonding or non-bonding, either by preventing bonding or by directing disulfide bonding between particular cysteine residues. Enzymes might also cleave "incorrect" bonding to enable the molecule to reorientate itself and assume the correct natural structure. Cysteine residues that are not internally bonded may be disulfide-bonded to free cysteine moieties. The three-dimensional structure of the molecule may also be such that random bonding or non-bonding of cysteine residues, either with each other or to free cysteines, does not substantially affect the biological structure of the protein molecule.

To synthesize a protein having the mammalian CRF-BP amino acid residue sequence by recombinant DNA, a double-stranded DNA chain which encodes CRF-BP might be synthetically constructed. Although it is nowadays felt that PCR techniques would be method of choice to produce DNA chains, a DNA chain encoding CRF-BP could be designed using certain particular codons that are more efficient for polypeptide expression in a certain type of organism, i.e. selection might employ those codons which are most efficient for expression in the type of organism which is to serve as the host for the recombinant vector. However, any correct set of codons will encode a desired product, although perhaps slightly less efficiently. Codon selection may also depend upon vector construction considerations; for example, it may be necessary to avoid placing a particular restriction site in the DNA chain if, subsequent to inserting the synthetic DNA chain, the vector is to be manipulated using the restriction enzyme that cleaves at such a site. Also, one should avoid placing restriction sites in the DNA chain if the host organism, which is to be transformed with the recombinant vector containing the DNA chain, is known to produce a restriction enzyme that would cleave at such a site within the DNA chain.

To assemble a synthetic CRF-BP-encoding DNA chain, oligonucleotides might be constructed by conventional procedures, such as those described in T. Maniatis et al., Molecular Cloning, A Laboratory Manual, second edition, Cold Spring Harbor Laboratory Press, New York (1989) (hereinafter, MCLM). Sense and antisense oligonucleotide chains, up to about 70 nucleotide residues long, are synthesized, preferably on automated synthesizers, such as the Applied Biosystem Inc. Model 380A DNA synthesizer. The oligonucleotide chains are constructed so that portions of the sense and antisense oligonucleotides overlap, associating with each other through hydrogen bonding between complementary base pairs and thereby forming double stranded chains, in most cases with gaps in the strands. Subsequently, the gaps in the strands are filled in, and oligonucleotides of each strand are joined end to end with nucleotide triphosphates in the presence of appropriate DNA polymerases and/or with ligases.

As an alternative to such stepwise construction of a synthetic DNA chain, the cDNA corresponding to CRF-BP that was cloned to deduce the complete structure of CRF-BP is used. As is well known, a cDNA library or an expression library is produced in a conventional manner by reverse transcription from messenger RNA (mRNA) from a suitable CRF-BP-producing cell line or tissue for the desired mammalian species. To select clones containing CRF-BP sequences, the hybridization probe obtained by PCR technology (or mixed probes which accommodate the degeneracy of the genetic code and correspond to a selected portion of the CRF-BP protein are produced) is used to identify clones containing such sequences. Screening of such an expression library with CRF-BP antibodies may also be used, either alone or in conjunction with hybridization probing, to identify or confirm the presence of CRF-BP-encoding DNA sequences in cDNA library clones which are expressing CRF-BP. Such techniques are taught, for example in MCLM, supra.

In addition to the CRF-BP-encoding sequences, a DNA chain should contain additional sequences depending upon vector construction considerations. Typically, a synthesized DNA chain has linkers at its ends to facilitate insertion into restriction sites within a cloning vector. A DNA chain may be constructed so as to encode the CRF-BP amino acid sequences as a portion of a fusion polypeptide; and if so, it will generally contain terminal sequences that encode amino acid residue sequences that serve as proteolytic processing sites, whereby the CRF-BP polypeptide may be proteolytically cleaved from the remainder of the fusion polypeptide. The terminal portions of the synthetic DNA chain may also contain appropriate start and stop signals.

Accordingly, a double-stranded CRF-BP-encoding DNA chain is constructed or modified with appropriate linkers for its insertion into a particular appropriate cloning vector. The cloning vector that is to be recombined to incorporate the DNA chain is selected appropriate to its viability and expression in a host organism or cell line, and the manner of insertion of the DNA chain depends upon factors particular to the host. For example, if the DNA chain is to be inserted into a vector for insertion into a prokaryotic cell, such as E. coli, the DNA chain will be inserted 3' of a promoter sequence, a Shine-Delgarno sequence (or ribosome binding site) that is within a 5' non-translated portion and an ATG start codon. The ATG start codon is appropriately spaced from the Shine-Delgarno sequence, and the encoding sequence is placed in correct reading frame with the ATG start codon. The cloning vector also provides a 3' non-translated region and a translation termination site. For insertion into a eukaryotic cell, such as a yeast cell or a cell line obtained from a higher animal, the CRF-BP-encoding oligonucleotide sequence is appropriately spaced from a capping site and in correct reading frame with an ATG start signal. The cloning vector also provides a 3' non-translated region and a translation termination site.

Prokaryotic transformation vectors, such as pBR322, pMB9, Col E1, pCR1, RP4 and lambda-phage, are available for inserting a DNA chain of the length which encodes CRF-BP with substantial assurance of at least some expression of the encoded polypeptide. Typically, such vectors are constructed or modified to have one or more unique restriction sites appropriately positioned relative to a promoter, such as the lac promoter. The DNA chain may be inserted with appropriate linkers into such a restriction site, with substantial assurance of production of CRF-BP in a prokaryotic cell line transformed with the recombinant vector. To assure proper reading frame, linkers of various lengths may be provided at the ends of the CRF-BP-encoding sequences. Alternatively, cassettes, which include sequences, such as the 5' region of the lac Z gene (including the operator, promoter, transcription start site, Shine-Delgarno sequence and translation initiation signal), the regulatory region from the tryptophane gene (trp operator, promoter, ribosome binding site and translation initiator), and a fusion gene containing these two promoters called the trp-lac or commonly called the Tac promoter are available into which the synthetic DNA chain may be conveniently inserted and then the cassette inserted into a cloning vector of choice.

Similarly, eukaryotic transformation vectors, such as, the cloned bovine papilloma virus genome, the cloned genomes of the murine retroviruses, and eukaryotic cassettes, such as the pSV-2 gpt system (described by Mulligan and Berg, Nature 277, 108-114, 1979), the Okayama-Berg cloning system (Mol. Cell Biol. 2, 161-170, 1982), and the expression cloning vector recently described by Genetics Institute (Science 228, 810-815, 1985), are available which provide substantial assurance of at least some expression of CRF-BP in the transformed eukaryotic cell line.

As previously mentioned, a convenient way to ensure production of CRF-BP or a protein of a similar length is to produce the protein initially as a segment of a gene-encoded fusion protein. In such case, the DNA chain is constructed so that the expressed protein has enzymatic processing sites flanking the CRF-BP amino acid residue sequences. A CRF-BP-encoding DNA chain may be inserted, for example, into the beta-galactosidase gene for insertion into E. coli, in which case, the expressed fusion protein is subsequently cleaved with proteolytic enzymes to release the CRF-BP from beta-galactosidase peptide sequences.

An advantage of inserting the CRF-BP-encoding sequence so that the CRF-BP sequence is expressed as a cleavable segment of a fusion protein, e.g. as the CRF-BP sequence fused within the beta-galactosidase peptide sequence, is that the endogenous protein into which the CRF-BP sequence is inserted is generally rendered non-functional, thereby facilitating selection for vectors encoding the fusion protein.

The CRF-BP protein may also be reproduced in yeast using known recombinant DNA techniques. For example, a plasmid containing CRF-BP (pCRF-BP), is amplified in a pCRF-BP-producing E. coli, clone, is isolated and is then cleaved with Eco RI and Sal I. This digested plasmid is electrophoresed on an agarose gel allowing for the separation and recovery of the amplified pCRF-BP insert. The insert is inserted into the plasmic pYEp, a shuttle vector which can be used to transform both E. coli, and Saccharomyces cerevisiae yeast. Insertion of the synthetic DNA chain at this point assures that the DNA sequence is under the control of a promoter, in proper reading frame from an ATG signal and properly spaced relative to a cap site. The shuttle vector is used to transform URA3, a strain of S. cerevisise yeast from which the oratate monophosphate decarboxylase gene is deleted.

The transformed yeast is grown in medium to attain log growth. The yeast is separated from its culture medium, and cell lysates are prepared. Pooled cell lysates are determined by RIA to be reactive with antibody raised against CRF-BP, demonstrating that a protein containing CRF-BP protein segment is expressed within the yeast cells. The production of CRF-BP can be carried out in both prokaryotic and eukaryotic cell lines to provide protein for biological and therapeutic use. While CRF-BP synthesis is easily demonstrated using either bacteria or yeast cell lines, the synthetic genes should be insertable for expression in cells of higher animals, such as mammalian tumor cells. Such mammalian cells may be grown, for example, as peritoneal tumors in host animals, and CRF-BP harvested from the peritoneal fluid.

Although the above examples demonstrate that CRF-BP can be synthesized through recombinant DNA techniques, the examples do not purport to have maximized CRF-BP production. It is expected that subsequent selection of more efficient cloning vectors and host cell lines will increase yields of CRF-BP. Known gene amplification techniques for both eukaryotic and prokaryotic cells may also be used to increase production of CRF-BP. Secretion of the gene-encoded protein from the host cell line into the culture medium is also considered to be an important factor in obtaining synthetic CRF-BP in large quantities.

The availability of such mammalian CRF-BP proteins permits their use to complex and neutralize CRF, and these proteins should be useful in the treatment of conditions which are caused by an overabundance of CRF, for example, during chronic stress or in the presence of a CRF-secreting tumor. Furthermore, CRF-BP's can be used to bind, sequester and/or detect CRF either by themselves or in conjunction with an antibody to CRF, using "two-site" methodology. The binding ability of CRF-BPs allows them to be used in an affinity chromatography column to purify hCRF. Moreover, administration of substantially pure monoclonal antibodies to CRF-BP have potential therapeutic applications to treat cases where it is desired to counteract the binding effect of CRF-BPs.

Substantially pure CRF-BP protein can be routinely obtained having significantly higher purity than CRF-BP that is present in crude extracts from mammalian serum. CRF-BP proteins constitute only minor constituents of normal mammalian serum, being present in only very impure form, relative to other native proteins also present. Because of the work involved and the low concentration in plasma, it would be impractical to prepare CRF-BP by purification from natural sources. Recombinant DNA techniques, for example, can be used to generate organisms or cell lines that produce the heterologous protein in significantly higher proportions, relative to total protein, in the cellular material and/or the secretions thereof--as compared to the proportions at which native CRF-BP are present. Because the starting material from which such synthetic CRF-BP proteins are isolated has a substantially greater concentration of the heterologous protein, available purification techniques can fairly simply produce more highly purified CRF-BP preparations in relatively copious amounts. Using appropriate isolation techniques, it is possible to routinely obtain CRF-BP proteins which are at least about 98% pure (by weight of total proteins) and which is herein referred to as substantially pure.

The protein should be administered under the guidance of a physician, and pharmaceutical compositions will usually contain the protein in conjunction with a conventional, pharmaceutically-acceptable carrier. For treatment, substantially pure synthetic CRF-BP or a nontoxic salt thereof, combined with a pharmaceutically acceptable carrier to form a pharmaceutical composition, is preferably administered parenterally to mammals, including humans, either intravenously, subcutaneously, intramuscularly, percutaneously, e.g. intranasally, or introcerebroventricularly; oral administration is possible with an appropriate carrier. The required dosage will vary with the particular treatment and with the duration of desired treatment; however, it is anticipated that dosages between about 10 micrograms and about 1 milligram per kilogram of body weight per day will be used for therapeutic treatment. Antibodies are administered in proportionately appropriate amounts in accordance with known practices in this art.

Examples of pharmaceutically acceptable nontoxic salts include acid addition salts and metal complexes, e.g., with zinc, iron or the like (which are broadly considered as salts for purposes of this application). Illustrative of such acid addition salts are hydrochloride, hydrobromide, sulphate, phosphate, maleate, acetate, citrate, benzoate, succinate, malate, ascorbate, tartrate and the like.

It may also be desirable to deliver CRF-BP over prolonged periods of time, for example, for periods of one week to one year from a single administration, and slow release, depot or implant dosage forms may be utilized. For example, a dosage form may contain a pharmaceutically acceptable non-toxic salt of the compound which has a low degree of solubility in body fluids, for example, an acid addition salt with the polybasic acid; a salt with a polyvalent metal cation; or combination of the two salts. A relatively insoluble salt may also be formulated in a gel, for example, an aluminum stearate gel. A suitable slow release depot formulation for injection may also contain CRF-BP or a salt thereof dispersed or encapsulated in a slow degrading, non-toxic or non-antigenic polymer such as a polylactic acid/polyglycolic acid polymer, for example, as described in U.S. Pat. No. 3,773,919. These compounds may also be formulated into silastic implants.

As previously indicated, administration of the CRF-BPs is effective to reduce high ACTH levels in mammals caused by excessive CRF. In this manner, the CRF-BPs are useful in treating the high cortisol levels associated with hypercortisolemia, Cushing's Disease, alcoholism, anorexia nervosa and similar diseases. Likewise, these CRF-BPs are considered to have utility in combatting pitiutary tumors that produce CRF--particularly in maintaining stability in the patient until such a tumor can be surgically removed. These proteins are also useful to treat abnormalities which occur during the later stages of pregnancies; for example, they can be used to reduce pregnancy-induced complications and increased CRF levels which can otherwise result in excessive release of ACTH. The IV administration of CRF-BPs may also be employed in certain instances to modulate blood pressure and thereby combat hypotension. CRF has been reported to be elevated in the plasma of some patients with preeclampsia (toxemia of pregnancy). If this increased level of CRF is clinically significant, then the CRF-BP could be useful in the therapeutic management of preeclampsia. More particularly, CRF is a known modulator or the immune system, and it is considered that the adminstration of the protein CRF-BP may be useful to locally treat arthritis and other similar ailments. CRF is known to have a number of biological effects on the pituitary, and accordingly, the CRF-BP proteins can be used to modulate the action of CRF on the pituitary. Furthermore, it is well known that CRF has a number of biological effects in the brain; therefore, it is considered that the CRF-BP proteins can be effectively used to modulate the action of CRF on the brain, particularly with respect to control of appetite, reproduction, growth, anxiety, depression, fever and metabolism, as well as the regulation of blood pressure, heart rate and blood flow.

In addition to administration parenterally or otherwise to mammals, it is expected that CRF-BPs will be an important diagnostic tool for monitoring changes within humans. Because in certain instances it is anticipated that the levels of CRF-BPs in the body will change along with changes in CRF levels, the employment of antibodies to CRF-BPs can be effectively employed in assays for monitoring such changes. Also, as earlier indicated, the antibodies can be used to purify the protein as well as to counteract the biological effect of CRF-BPs when administered in vivo. Administration of the antibodies for this purpose would be carried out along the lines and in amounts generally known in this art, and more particularly along the lines indicated hereinbefore with respect to administration of the protein itself.

For purposes of this application, mammalian CRF-BP proteins should be considered to constitute proteins having the amino acid residue sequences set forth hereinbefore as well as naturally occurring amino acid sequence variants of other mammalian species and fragments of the foregoing having equivalent biological activity. Unless otherwise stated hereinbefore, all percentages are volume percents.

Although the invention has been described with regard to its preferred embodiments, which constitute the best mode presently known to the inventors, it should be understood that various changes and modifications as would be obvious to one having the ordinary skill in this art may be made without departing from the scope of the invention which is set forth in the claims appended hereto. For example, biologically active fragments of such proteins, shortened at the C-terminus or at the N-terminus or at both termini, can be employed instead of the entire protein to have the same biological effect of inactivating CRF.

## Claims

1. DNA encoding CRF-binding protein (CRF-BP), said DNA being DNA as defined in one of integers A, B, C and D below:-
A. recombinant DNA comprising a DNA sequence encoding the AA sequence of an human CRF (hCRF) binding protein (hCRF-BP) which binds to hCRF to render inactive said hCRF, said DNA sequence being hybridizable to the DNA defined in integer B below and being obtainable by the following specific procedures:-
(i) purifying hCRF-BP from human plasma by a purification process comprised of:-
(a) elution of the human plasma through an hCRF-Sepharose affinity column,
(b) gel filtration of the eluting fraction,
(c) micropreparative SDS gel electrophoresis,
(d) transfer of the resulting bands to nitrocellulose, and
(e) excising the major protein band;
(ii) obtaining two tryptic fragments identified by the AA sequences set forth in integer (iii) below from said excised protein band by a process comprised of:-
(a) trypsinising the excised protein band by treatment with typsin, and
(b) recovering a fraction comprising the aforesaid two tryptic fragments from the supernatant formed in the trypsinising treatment, followed by RP-HPLC resolution thereof
(iii) advancing to an olignucleotide primer preparation stage two polypeptides, respectively having the AA sequences set forth below, said polypeptides being the resolved tryptic fragments obtained from step (ii)(b) aforesaid:-
Glu-Ala-Asp-Tyr-Asp-Pro-Phe-Leu-Leu-Phe-Ser-Ala-Asn-Leu-Lys
and
Ser-Ser-Gln-Asn-Val-Ala-Met-Ile-Phe-Phe-Arg
(iv) forming the two sets of degenerate oligonucleotide primers defined below and corresponding to portions of the aforesaid tryptic fragment AA sequences:- 
(Asp) (Tyr) Asp Pro (Phe) (Leu) (Leu) (Phe)
GA (T/C) TA(T/C) GAT CCN TT (T/C) (C/T) TN (C/T) TN TT (T/C)

(Ser) (Ala) Asn
(T/A) (C/G) N GCN AAC; and

(Gln) (Asn) ValAlaMetIle(Phe) Phe
CA (A/G) AA (T/C) GTNGCNATGATNTT (C/T) TTC
(v) performing multiple cycles of PCR using DNA from a human adult liver cDNA library as a template to obtain a DNA fragment encoding a peptide containing said two AA sequences, and
(vi) using said DNA fragment as a probe to screen said human liver cDNA library to obtain DNA encoding human CRF-BP containing said two AA sequences;
B. recombinant DNA comprising a DNA sequence encoding the AA sequence of a rat CRF (rCRF) binding protein (rCRF-BP) which binds to hCRF to render inactive said hCRF, said rCRF-BP having AA SEQ ID NO: 3;
C. recombinant DNA comprising a DNA sequence encoding the AA sequence of a homolog of said hCRF-BP or of said rCRF-BP which homolog is native to another mammalian species and binds to hCRF to render inactive said hCRF ; and
D. recombinant DNA comprising a DNA sequence encoding the AA sequence of a CRF-binding AA fragment of hCRF-BP, rCRF-BP or hCRF-BP or rCRF-BP homolog as defined in integer C above.

2. The DNA as claimed in Claim 1 wherein there are no interruptions by introns.

3. A DNA as claimed in Claim 1 having nucleotide sequence SEQ ID NO:2, or a homologous naturally occurring nucleotide sequence of another mammalian species which is hybridizable thereto and which encodes a CRF-binding protein as defined in Claim 1.

4. A replicable recombinant DNA expression vector which includes DNA as claimed in Claim 1, said vector being capable of expressing the DNA in a microorganism or cell culture wherein said vector is inserted, and wherein, upon expression, a protein is produced which is a protein having the amino acid sequence which is a translation of said DNA or which is a shortened protein whose amino acid sequence is an N-terminally shortened version of said first-mentioned amino acid sequence which shortened protein biologically inactivates hCRF.

5. Recombinant host cells transformed with a vector as claimed in Claim 4.

6. A method of producing a CRF-binding protein which method comprises culturing host cells as claimed in Claim 5 under conditions which permit the expression of the DNA and recovering the protein produced, wherein the host cells are either bacteria or mammalian cells.

7. A microorganism transformed with a vector as claimed in Claim 4, said microorganism being capable of expressing the DNA encoding the CRF-binding protein.

8. A cell culture capable of expressing DNA encoding a CRF-binding protein, which cell culture is obtained by transforming a cell line with a vector as claimed in Claim 4.

9. A method of producing a CRF-binding protein, which method comprises growing a cell culture as claimed in Claim 8 under conditions permitting expression of the DNA of said vector and recovering the protein produced.

10. A recombinant protein having the following characteristics:-
(i) it complexes to corticotropin releasing factor ;
(ii) it contains an AA sequence as defined below:-
(a) an AA sequence as encoded by hCRF-BP DNA as claimed in integer A of Claim 1;
(b) an AA sequence of rCRF-BP as defined in integer B of Claim 1, namely AA SEQ ID NO: 3;
(c) an AA sequence of an hCRF-BP or rCRF-BP homolog as defined in integer C of Claim 1; or
(d) an AA sequence as defined in integer D of Claim 1 of a CRF-binding AA fragment
(iii) it has a purity of at least about 98%; and
(iv) it binds to hCRF so as to effect biological inactivity thereof.

11. Use of DNA as claimed in any one of Claims 1 to 3 for the production of protein encoded thereby.

## Patentansprüche

1. DNA-codierendes CRF-bindendes Protein (CRF-BP), wobei die DNA wie nachfolgend in A, B, C oder D definiert ist:
A. rekombinante DNA umfassend eine DNA-Sequenz, die die AA-Sequenz eines Human-CRF-(hCRF)-bindenden Proteins (hCRF-BP) codiert, welches zur Inaktivierung von hCRF an hCRF bindet, wobei die DNA-Sequenz an die folgend in B definierte DNA hybridisierbar und durch die folgenden spezifischen Arbeitsgänge erhältlich ist:
(i) Reinigen von hCRF-BP aus Humanplasma mittels eines Reinigungsverfahrens umfassend:
(a) Elution des menschlichen Plasmas durch eine hCRF-Sepharose-Affinitätssäule,
(b) Gelfileration der eluierenden Fraktion,
(c) mikropräparative SDS-Gelelektrophorese,
(d) Übertragung der resultierenden Banden auf Nitrocellulose, und
(e) Excision der Haupt-Proteinbande;
(ii) Erhalten zweier durch die nachfolgend in (iii) dargelegten AA-Sequenzen identifizierter tryptischer Fragmente aus der ausgeschnittenen Proteinbande duch ein Verfahren umfassend:
(a) Trypsinieren der ausgeschnittenen Proteinbande durch Behandeln mit Trypsin und
(b) Gewinnen einer die zwei vorgenannten tryptischen Fragmente umfassenden Fraktion aus dem bei der Trypsinisierungsbehandlung gebildeten Überstand, gefolgt von RP-HPLC-Auflösung derselben
(iii) Weiterleiten zweier die jeweilige nachfolgend dargelegte AA-Sequenz aufweisender Polypeptide zu einer Oligonucleotidprimerherstellungsstufe, wobei die Polypeptide die aus obigem Schritt (ii) (b) erhaltenen aufgelösten tryptischen Fragmente sind:
Glu-Ala-Ala-Asp-Tyr-Asp-Pro-Phe-Leu-Leu-Phe-Ser-Ala-Asn-Leu-Lys
bzw.
Ser-Ser-Gln-Asn-Val-Ala-Met-Ile-Phe-Phe-Arg
(iv) Bilden der beiden nachstehend definierten Gruppen von degenerierten Oligonucleotidprimern, die Teilen der vorgenannten AA-Sequenzen der tryptischen Fragmente entsprechen: 
(Asp)    (Tyr)    Asp Pro (Phe) (Leu)    (Leu)    (Phe)
GA (T/C) TA(T/C) GAT CCN TT (T/C) (C/T) TN (C/T) TN TT (T/C)

(Ser)    (Ala) Asn
(T/A) (C/G) N GCN AAC; und

(Gln)    (Asn)    ValAlaMetIle(Phe)    Phe
CA (A/G) AA (T/C) GTNGCNATGATNTT (C/T) TTC
(v) Ausführen von mehrfachen PCR-Zyklen unter Verwendung von DNA aus einer menschliche-Erwachsenen-Leber-cDNA-Bibliothek als Matrize, um ein DNA-Fragment zu erhalten, das ein die beiden AA-Sequenzen enthaltendes Peptid codiert, und
(vi) Verwenden des DNA-Fragments als Sonde zum Durchmustern der menschliche-Leber-cDNA-Bibliothek, um eine DNA zu erhalten, die das die beiden AA-Sequenzen enthaltende menchliche CRF-BP codiert;
B. rekombinante DNA umfassend eine DNA-Sequenz, die die AA-Sequenz eines Ratten-CRF-(rCRF)-bindenden Proteins (rCRF-BP) codiert, welches zur Inaktivierung von hCRF an hCRF bindet, wobei das rCRF-BP AA SEQ ID NO: 3 aufweist;
C. rekombinante DNA umfassend eine DNA-Sequenz, die die AA-Sequenz eines hCRF-BP-Homologs oder eines rCRF-BP-Homologs codiert, welches Homolog für eine andere Säugerart nativ ist und zur Inaktivierung von hCRF an hCRF bindet; und
D. rekombinante DNA umfassend eine DNA-Sequenz, die die AA-Sequenz eines CRF-bindenden AA-Fragments von hCRF-BP, rCRF-BP oder eines hCRF-BP- oder rCRF-BP-Homologs wie zuvor in C definiert codiert.

2. DNA nach Anspruch 1, worin es keine Unterbrechungen durch Introns gibt.

3. DNA nach Anspruch 1 mit der Nucleotidsequenz SEQ ID NO:2 oder homologe natürlich vorkommende Nucleotidsequenz einer anderen Säugerart, die daran hybridisierbar ist und ein CRF-bindendes Protein nach Anspruch 1 codiert.

4. Replizierbarer rekombinanter DNA-Expressionsvektor, der eine DNA nach Anspuch 1 enthält, welcher Vektor die DNA in einem Mikroorganismus oder in einer Zellkultur exprimieren kann, worin der Vektor inseriert ist, und worin nach der Expression ein Protein erzeugt wird, das ein Protein mit der Aminosäuresequenz ist, die eine Translation dieser DNA ist, oder das ein verkürztes Protein ist, dessen Aminosäuresequenz eine N-terminal verkürzte Version der erstgenannten Aminosäuresequenz ist, welches verkürzte Protein hCRF biologisch inaktiviert.

5. Rekombinante Wirtszellen, die mit einem Vektor nach Anspruch 4 transformiert sind.

6. Verfahren zur Herstellung eines CRF-bindenden Proteins, welches Verfahren das Kultivieren von Wirtszellen nach Anspruch 5 unter Bedingungen umfaßt, die die Expression der DNA und die Gewinnung des erzeugen Proteins gestatten, worin die Wirtszellen entweder Bakterien oder Säugerzellen sind.

7. Mikroorganismus, der mit einem Vektor nach Anspruch 4 transformiert ist, welcher Mikroorganismus die das CRF-bindende Protein codierende DNA exprimieren kann.

8. Zellkultur, die die ein CRF-bindendes Protein codierende DNA exprimieren kann, welche Zellkultur durch Transformation einer Zelllinie mit einem Vektor nach Anspruch 4 erhalten wird.

9. Verfahren zur Herstellung eines CRF-bindenden Proteins, welches Verfahren das Züchten einer Zellkultur nach Anspruch 8 unter Bedingungen umfaßt, die die Expression der DNA dieses Vektors und die Gewinnung des erzeugten Proteins gestatten.

10. Rekombinantes Protein mit den folgenden Merkmalen:
(i) es komplexiert zum Corticotropin-Freisetzungsfaktor;
(ii) es enthält eine AA-Sequenz wie nachstehend definiert:
(a) eine AA-Sequenz wie von der unter A. von Anspruch 1 definierten hCRF-BP-DNA codiert;
(b) eine AA-Sequenz von rCRF-BP wie unter B. von Anspruch 1 definiert, nämlich AA SEQ ID NO: 3;
(c) eine AA-Sequenz eines hCRF-BP- oder rCRF-BP-Homologs wie unter C. von Anspruch 1 definiert; oder
(d) eine AA-Sequenz eines CRF-bindenden AA-Fragments wie unter D. von Anspruch 1 definiert;
(iii) es hat eine Reinheit von zumindest etwa 98 %; und
(iv) es bindet an hCRF, um die biologische Inaktivität desselben zu bewirken.

11. Verwendung der DNA nach einem der Ansprüche 1 bis 3 zur Herstellung von dadurch codiertem Protein.

## Revendications

1. ADN codant pour la protéine de liaison du facteur de libération de corticotrophine (CRF) (PL-CRF), ledit ADN étant un ADN tel que défini ci-après dans l'un des points A, B, C et D:
A. ADN recombinant comprenant une séquence d'ADN codante pour la séquence AA d'une protéine de liaison de CRF humaine (CRFh) (PL-CRFh) qui se lie au CRFh pour rendre celui-ci inactif, ladite séquence d'ADN pouvant être hybridée avec l'ADN défini en B ci-dessous et pouvant être obtenue au moyen des modes opératoires spécifiques suivants :
(i) purifier une PL-CRFh à partir de plasma humain au moyen d'un procédé de purification comprenant :
(a) une élution du plasma humain à travers une colonne d'affinité de CRFh-Sepharose,
(b) une filtration sur gel de la fraction éluée,
(c) une électrophorèse micropréparative sur gel de SDS,
(d) le transfert des bandes résultantes sur de la nitrocellulose et
(e) l'excision de la bande protéique principale;
(ii) obtenir deux fragments trypsiques identifiés par les séquences AA indiquées ci-après en (iii), à partir de ladite bande protéique excisée, au moyen d'un procédé comprenant :
(a) la trypsination de la bande protéique excisée au moyen d'un traitement à la trypsine, et
(b) la récupération d'une fraction comprenant les deux fragments trypsiques susmentionnés à partir du surnageant formé pendant le traitement de trypsination, suivie d'une séparation de ceux-ci par HPLC en phases inversées ;
(iii) amener à une étape de préparation d'amorce oligonucléotidique deux polypeptides ayant respectivement les séquences AA indiquées ci-après, lesdits polypeptides étant les fragments trypsiques séparés obtenus pendant l'étape (ii)(b) susmentionnée :
Glu-Ala-Ala-Asp-Tyr-Asp-Pro-Phe-Leu-Leu-Phe-Ser-Ala-Asn-Leu-Lys
et
Ser-Ser-Gln-Asn-Val-Ala-Met-Ile-Phe-Phe-Arg
(iv) former les deux groupes d'amorces oligonucléotidiques dégénérées définies ci-après et correspondant à des parties des séquences susmentionnées AA des fragments trypsiques : 
(Asp) (Tyr) Asp Pro (Phe) (Leu) (Leu) (Phe)
GA (T/C) TA(T/C) GAT CCN TT (T/C) (C/T) TN (C/T) TN TT (T/C)

(Ser) (Ala) Asn
(T/A) (C/G) N GCN AAC ; et

(Gln) (Asn) ValAlaMetIle(Phe) Phe
CA (A/G) AA (T/C) GTNGCNATGATNTT (C/T) TTC
(v) effectuer des cycles multiples d'amplification en chaîne par polymérase (ACP) en utilisant un ADN provenant d'une banque d'ADNc de foie humain adulte en tant que matrice pour obtenir un fragment d'ADN codant pour un peptide contenant lesdites deux séquences AA, et
(vi) utiliser ledit fragment d'ADN en tant que sonde pour dépister ladite banque d'ADNc de foie humain pour obtenir un ADN codant pour la PL-CRF humaine
contenant lesdites deux séquences AA ;
B. ADN recombinant comprenant une séquence d'ADN codante pour la séquence AA d'une protéine de liaison de CRF de rat (CRFr) (PL-CRFr) qui se lie au CRFh pour rendre celui-ci inactif, ladite PL-CRFr ayant la séquence AA ID NO: 3 ; C. ADN recombinant comprenant une séquence d'ADN codante pour la séquence AA d'un homologue de ladite PL-CRFh ou de ladite PL-CRFr, ledit homologue étant natif par rapport à une autre espèce mammifère et se liant au CRFh pour rendre celui-ci inactif ; et
D. ADN recombinant comprenant une séquence d'ADN codante pour la séquence AA d'un fragment AA de liaison du CRF, de PL-CRFh, de PL-CRFr ou d'un homologue de PL-CRFh ou de PL-CRFr tels que définis ci-dessus en C.

2. ADN selon la revendication 1, dans lequel il n'y a pas d'interruptions par des introns.

3. ADN selon la revendication 1, ayant une séquence nucléotidique SEQ ID NO:2, ou une séquence nucléotidique homologue apparaissant naturellement d'une autre espèce mammifère, qui peut être hybridée avec celle-ci et qui code pour une protéine de liaison de CRF telle que définie dans la revendication 1.

4. Vecteur d'expression d'ADN recombinant réplicable qui comprend l'ADN suivant la revendication 1, ledit vecteur étant apte à l'expression de l'ADN dans une culture de micro-organismes ou une culture cellulaire dans laquelle ledit vecteur est inséré, et dans laquelle, par expression, est produite une protéine qui consiste en une protéine ayant la séquence d'aminoacides qui est une traduction dudit ADN ou qui est une protéine raccourcie dont la séquence d'aminoacides est une variante raccourcie au niveau N-terminal de ladite première séquence d'aminoacides mentionnée, protéine raccourcie qui inactive biologiquement le CRFh.

5. Cellules hôtes recombinantes transformées avec un vecteur selon la revendication 4.

6. Procédé pour la production d'une protéine de liaison de CRF, procédé qui comprend les étapes consistant à cultiver des cellules hôtes selon la revendication 5 dans des conditions qui permettent l'expression de l'ADN et à recueillir la protéine produite, dans lequel les cellules hôtes sont des cellules bactériennes ou cellules de mammifères.

7. Micro-organisme transformé avec un vecteur selon la revendication 4, ledit micro-organisme étant capable d'exprimer l'ADN codant pour la protéine de liaison de CRF.

8. Culture cellulaire capable d'exprimer l'ADN codant pour une protéine de liaison de CRF, ladite culture cellulaire étant obtenue par transformation d'une lignés cellulaire avec un vecteur selon la revendication 4.

9. Procédé pour la production d'une protéine de liaison de CRF, procédé qui comprend les étapes consistant à multiplier une culture cellulaire selon la revendication 8 dans des conditions permettant l'expression de l'ADN dudit vecteur, et à recueillir la protéine produite.

10. Protéine recombinante ayant les caractéristiques suivantes :
(i) elle subit une complexation avec le facteur de libération de corticotrophine ;
(ii) elle contient une séquence AA répondant à la définition ci-dessous :
(a) une séquence AA codée par l'ADN de PL-CRFh selon le point (A) de la revendication 1 ;
(b) une séquence AA de PL-CRFh telle que définie dans le point B de la revendication 1, à savoir la SEQ AA ID N° 3
(c) une séquence AA d'une PL-CRFh ou d'un homologue de PL-CRFh tel que défini dans le point C de la revendication 1 ; ou
(d) une séquence AA telle que définie dans le point D de la revendication 1 d'un fragment AA de liaison de CRF.
(iii) elle a une pureté d'au moins environ 98 % ; et
(iv) elle se lie au CRFh pour le rendre biologiquement. inactif.

11. Utilisation de l'ADN selon l'une quelconque des revendications 1 à 3 pour la production d'une protéine codée par cet ADN.


SEQUENCE LISTING

(1) GENERAL INFORMATION:

(i) APPLICANT: Potter, Ellen
Behan, Dominic P
Fischer, Wolfgang H
Linton, Elizabeth A
Lowry, Philip J
Vale Jr, Wylie W

(ii) TITLE OF INVENTION: CRF Binding Protein

(iii) NUMBER OF SEQUENCES: 4

(iv) CORRESPONDENCE ADDRESS:
(A) ADDRESSEE: Fitch, Even, Tabin & Flannery
(B) STREET: 135 South LaSalle Street, Suite 900
(C) CITY: Chicago
(D) STATE: Illinois
(E) COUNTRY: USA
(F) ZIP: 60603

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.24

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 07/641,341
(B) FILING DATE: 15-JAN-1991

(viii) ATTORNEY/AGENT INFORMATION:
(A) NAME: Watt, Phillip H.
(B) REGISTRATION NUMBER: 25,939
(C) REFERENCE/DOCKET NUMBER: 50843PCT

(ix) TELECOMMUNICATION INFORMATION:
(A) TELEPHONE: (312)372-7842
(B) TELEFAX: (312)372-7848

(2) INFORMATION FOR SEQ ID NO:1:

(i) SEQUENCE CHARACTERISTICS:
(A) LENGTH: 322 amino acids
(B) TYPE: amino acid
(D) TOPOLOGY: linear

(ii) MOLECULE TYPE: protein

(ix) FEATURE:
(A) NAME/KEY: Protein
(B) LOCATION: join(25..298)
(D) OTHER INFORMATION:

(xi) SEQUENCE DESCRIPTION: SEQ ID NO:1:

Met Ser Pro Asn Phe Lys Leu Gln Cys His Phe Ile Leu Ile Phe Leu
-20 -15 -10

Thr Ala Leu Arg Gly Glu Ser Arg Tyr Leu Glu Leu Arg Glu Ala Ala
-5 1 5

Asp Tyr Asp Pro Phe Leu Leu Phe Ser Ala Asn Leu Lys Arg Asp Val
10 15 20

Ala Gly Glu Gln Pro Tyr Arg Arg Ala Leu Arg Cys Leu Asp Met Leu
25 30 35 40

Ser Leu Gln Gly Gln Phe Thr Phe Thr Ala Asp Arg Pro Gln Leu His
45 50 55

Cys Ala Ala Phe Phe Ile Ser Glu Pro Glu Glu Phe Ile Thr Ile His
60 65 70

Tyr Asp Gln Val Ser Ile Asp Cys Gln Gly Gly Asp Phe Leu Lys Val
75 80 85

Phe Asp Gly Trp Ile Leu Lys Gly Glu Lys Phe Pro Ser Ser Gln Asp
90 95 100

His Pro Leu Pro Ser Ala Glu Arg Tyr Ile Asp Phe Cys Glu Ser Gly
105 110 115 120

Leu Ser Arg Arg Ser Ile Arg Ser Ser Gln Asn Val Ala Met Ile Phe
125 130 135

Phe Arg Val His Glu Pro Gly Asn Gly Phe Thr Leu Thr Ile Lys Thr
140 145 150

Asp Pro Asn Leu Phe Pro Cys Asn Val Ile Ser Gln Thr Pro Asn Gly
155 160 165

Lys Phe Thr Leu Val Val Pro His Gln His Arg Asn Cys Ser Phe Ser
170 175 180

Ile Ile Tyr Pro Val Val Ile Lys Ile Ser Asp Leu Thr Leu Gly His
185 190 195 200

Val Asn Gly Leu Gln Leu Lys Lys Ser Ser Ala Gly Cys Glu Gly Ile
205 210 215

Gly Asp Phe Val Glu Leu Leu Glu Gly Thr Gly Leu Asp Pro Ser Lys
220 225 230

Met Thr Pro Leu Ala Asp Leu Cys Tyr Pro Phe His Gly Pro Ala Gln
235 240 245

Met Lys Val Gly Cys Asp Asn Thr Val Val Arg Met Val Ser Ser Gly
250 255 260

Lys His Val Asn Arg Val Thr Phe Glu Tyr Arg Gln Leu Glu Pro Tyr
265 270 275 280

Glu Leu Glu Asn Pro Asn Gly Asn Ser Ile Gly Glu Phe Cys Leu Ser
285 290 295

Gly Leu

(2) INFORMATION FOR SEQ ID NO:2:

(i) SEQUENCE CHARACTERISTICS:
(A) LENGTH: 1248 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 47..1015
(D) OTHER INFORMATION:
(ix) FEATURE:
(A) NAME/KEY: mat_peptide
(B) LOCATION: 119..322
(D) OTHER INFORMATION:

(xi) SEQUENCE DESCRIPTION: SEQ ID NO:2:

GGACCTCCGG AGCAGACAGC ACAGCAGCTG CAGAGGCAAG GCCAGC ATG TCG CCC 55
Met Ser Pro
-24

AAC TTC AAA CTT CAG TGT CAC TTC ATT CTC ATC TTC CTG ACG GCT CTA 103
Asn Phe Lys Leu Gln Cys His Phe Ile Leu Ile Phe Leu Thr Ala Leu
-20 -15 -10

AGA GGG GAA AGC CGG TAC CTA GAG CTG AGG GAA GCG GCG GAC TAC GAT 151
Arg Gly Glu Ser Arg Tyr Leu Glu Leu Arg Glu Ala Ala Asp Tyr Asp
-5 1 5 10

CCT TTC CTG CTC TTC AGC GCC AAC CTG AAG CGG GAC GTG GCT GGG GAG 199
Pro Phe Leu Leu Phe Ser Ala Asn Leu Lys Arg Asp Val Ala Gly Glu
15 20 25

CAG CCG TAC CGC CGC GCT CTG CGG TGC CTG GAC ATG CTG AGC CTC CAG 247
Gln Pro Tyr Arg Arg Ala Leu Arg Cys Leu Asp Met Leu Ser Leu Gln
30 35 40

GGC CAG TTC ACC TTC ACC GCC GAC CGG CCG CAG CTG CAC TGC GCA GCC 295
Gly Gln Phe Thr Phe Thr Ala Asp Arg Pro Gln Leu His Cys Ala Ala
45 50 55

TTC TTC ATC AGC GAG CCC GAG GAG TTC ATT ACC ATC CAC TAC GAC CAG 343
Phe Phe Ile Ser Glu Pro Glu Glu Phe Ile Thr Ile His Tyr Asp Gln
60 65 70 75

GTC TCC ATC GAC TGT CAG GGC GGC GAC TTC CTG AAG GTA TTT GAT GGT 391
Val Ser Ile Asp Cys Gln Gly Gly Asp Phe Leu Lys Val Phe Asp Gly
80 85 90

TGG ATT CTC AAG GGG GAG AAG TTC CCC AGT TCC CAG GAT CAT CCT CTC 439
Trp Ile Leu Lys Gly Glu Lys Phe Pro Ser Ser Gln Asp His Pro Leu
95 100 105

CCC TCA GCT GAG CGG TAC ATA GAT TTC TGT GAG AGT GGT CTT AGC AGG 487
Pro Ser Ala Glu Arg Tyr Ile Asp Phe Cys Glu Ser Gly Leu Ser Arg
110 115 120

AGG AGC ATC AGA TCT TCC CAG AAT GTG GCC ATG ATC TTC TTC CGA GTC 535
Arg Ser Ile Arg Ser Ser Gln Asn Val Ala Met Ile Phe Phe Arg Val
125 130 135

CAT GAA CCA GGA AAT GGA TTC ACA TTA ACC ATA AAG ACA GAC CCC AAC 583
His Glu Pro Gly Asn Gly Phe Thr Leu Thr Ile Lys Thr Asp Pro Asn
140 145 150 155

CTC TTT CCT TGC AAT GTC ATT TCT CAG ACT CCA AAT GGA AAG TTT ACC 631
Leu Phe Pro Cys Asn Val Ile Ser Gln Thr Pro Asn Gly Lys Phe Thr
160 165 170

CTG GTA GTT CCA CAC CAG CAT CGA AAC TGC AGC TTC TCC ATA ATT TAT 679
Leu Val Val Pro His Gln His Arg Asn Cys Ser Phe Ser Ile Ile Tyr
175 180 185

CCT GTG GTG ATC AAA ATA TCT GAT CTT ACC CTG GGA CAC GTA AAT GGT 727
Pro Val Val Ile Lys Ile Ser Asp Leu Thr Leu Gly His Val Asn Gly
190 195 200

CTT CAG TTA AAG AAA TCC TCA GCA GGT TGC GAG GGA ATA GGA GAC TTT 775
Leu Gln Leu Lys Lys Ser Ser Ala Gly Cys Glu Gly Ile Gly Asp Phe
205 210 215

GTG GAG CTG CTG GAG GGA ACT GGA TTG GAC CCT TCC AAG ATG ACG CCT 823
Val Glu Leu Leu Glu Gly Thr Gly Leu Asp Pro Ser Lys Met Thr Pro
220 225 230 235

TTA GCT GAT CTC TGC TAC CCC TTT CAT GGC CCG GCC CAG ATG AAA GTT 871
Leu Ala Asp Leu Cys Tyr Pro Phe His Gly Pro Ala Gln Met Lys Val
240 245 250

GGC TGT GAC AAC ACT GTG GTG CGC ATG GTC TCC AGT GGA AAA CAC GTA 919
Gly Cys Asp Asn Thr Val Val Arg Met Val Ser Ser Gly Lys His Val
255 260 265

AAT CGT GTG ACT TTT GAG TAT CGT CAG CTG GAG CCG TAC GAG CTG GAA 967
Asn Arg Val Thr Phe Glu Tyr Arg Gln Leu Glu Pro Tyr Glu Leu Glu
270 275 280

AAC CCA AAT GGA AAC AGT ATC GGG GAA TTC TGT TTG TCT GGT CTT TGA 1015
Asn Pro Asn Gly Asn Ser Ile Gly Glu Phe Cys Leu Ser Gly Leu .
285 290 295

ATAACCAACC CAGTGATTTA CATGCTGATA GCTAAGTGAG TTTTTAATGG CCATTGTGTA 1075

TGATTTTGAT GCACAACTAG TTAAAAGCCT TTCATACCAG TCAGTATTTC CCAGCCTTGA 1135

GCGCACGCAC ACACCACACA CATACACACA CGCATTATTT TTGTTACTTT GCTTCTTTTT 1195

ATGTTTGTAA TCTGTAAATG AACACATGGC AGAAAATAAC CCTGATTGGT AGG 1248

(2) INFORMATION FOR SEQ ID NO:3:

(i) SEQUENCE CHARACTERISTICS:
(A) LENGTH: 322 amino acids
(B) TYPE: amino acid
(D) TOPOLOGY: linear

(ii) MOLECULE TYPE: protein

(ix) FEATURE:
(A) NAME/KEY: Protein
(B) LOCATION: join(25..298)
(D) OTHER INFORMATION:

(xi) SEQUENCE DESCRIPTION: SEQ ID NO:3:

Met Ser Pro Asn Phe Lys Leu Gln Cys His Phe Thr Leu Ile Leu Leu
-20 -15 -10

Thr Ala Leu Arg Gly Glu Ser Arg Tyr Leu Glu Val Gln Glu Ala Ala
-5 1 5

Val Tyr Asp Pro Phe Leu Leu Phe Ser Ala Asn Leu Lys Arg Asn Leu
10 15 20

Ala Glu Glu Gln Pro Tyr Arg Arg Ala Leu Arg Cys Leu Asp Met Leu
25 30 35 40

Ser Leu Pro Gly Gln Phe Thr Phe Thr Ala Asp Gln Pro Gln Leu His
45 50 55

Cys Ala Ala Phe Phe Ile Gly Glu Pro Glu Glu Phe Ile Thr Ile His
60 65 70

Phe Asp Leu Val Ser Ile Asp Cys Gln Gly Gly Asp Phe Leu Lys Val
        75                  80                  85

Phe Asp Gly Trp Ile Leu Lys Gly Glu Lys Phe Pro Ser Ser Gln Asp
    90                  95                  100

His Pro Leu Pro Thr Arg Glu Arg Tyr Thr Asp Phe Cys Glu Ser Gly
105                 110                 115                 120

Leu Thr Arg Arg Ser Val Thr Ser Ser Gln Asn Val Ala Met Val Phe
                125                 130                 135

Phe Arg Val His Glu Pro Gly Asn Gly Phe Thr Ile Thr Ile Lys Thr
            140                 145                 150

Asp Pro Asn Leu Phe Pro Cys Asn Ile Ile Ser Gln Thr Pro Ser Gly
        155                 160                 165

Arg Phe Ala Leu Val Val Pro Tyr Gln His Gln Asn Cys Ser Phe Ser
    170                 175                 180

Ile Ile Tyr Pro Val Thr Ile Lys Ile Ser Asp Leu Ala Leu Gly His
185                 190                 195                 200

Leu His Gly Leu Gln Leu Lys Lys Pro Ala Ala Gly Cys Gly Gly Thr
                205                 210                 215

Gly Asp Phe Val Glu Leu Leu Gly Gly Thr Gly Leu Asp Thr Ser Lys
            220                 225                 230

Met Met Leu Leu Val Asp Leu Cys Tyr Pro Phe His Gly Pro Ala Gln
        235                 240                 245

Met Lys Ile Ser Cys Asp Asn Ala Val Val Arg Met Val Ser Ser Gly
    250                 255                 260

Lys His Met Asn Arg Val Thr Phe Glu Tyr Arg Gln Leu Glu Pro Leu
265                 270                 275                 280

Glu Leu Glu Thr Ser Thr Arg Asn Ser Ile Pro Glu Tyr Cys Leu Ser
                285                 290                 295

Ser Leu

(2) INFORMATION FOR SEQ ID NO:4:

    (i) SEQUENCE CHARACTERISTICS:
        (A) LENGTH: 1095 base pairs
        (B) TYPE: nucleic acid
        (C) STRANDEDNESS: single
        (D) TOPOLOGY: linear
   (ii) MOLECULE TYPE: cDNA

(ix) FEATURE:
(A) NAME/KEY: CDS
(B) LOCATION: 118..1086
(D) OTHER INFORMATION:
(ix) FEATURE:
(A) NAME/KEY: mat_peptide
(B) LOCATION: 190..1086
(D) OTHER INFORMATION:

(xi) SEQUENCE DESCRIPTION: SEQ ID NO:4:

AAAGAGACCC AGGAAAGGAC CCTAGCAGCT TCGAGTTCTC AGTGTGGGCG AAGGCGAGGG 60

AAGAAACGCC TAAGATCTCC GCAGCCGAGC TCACCAGCTG CAGACACAAG GCCAGCC 117

ATG TCA CCG AAC TTC AAA CTC CAA TGC CAC TTC ACT CTG ATC CTC CTG 165
Met Ser Pro Asn Phe Lys Leu Gln Cys His Phe Thr Leu Ile Leu Leu
-24 -20 -15 -10

ACA GCT CTA AGG GGA GAG AGC CGC TAC CTA GAG GTG CAA GAA GCC GCA 213
Thr Ala Leu Arg Gly Glu Ser Arg Tyr Leu Glu Val Gln Glu Ala Ala
-5 1 5

GTC TAC GAC CCT TTC CTG CTT TTC AGC GCC AAT TTG AAG CGG AAC CTG 261
Val Tyr Asp Pro Phe Leu Leu Phe Ser Ala Asn Leu Lys Arg Asn Leu
10 15 20

GCA GAG GAG CAG CCC TAC CGA CGG GCT CTG CGC TGC CTG GAC ATG CTG 309
Ala Glu Glu Gln Pro Tyr Arg Arg Ala Leu Arg Cys Leu Asp Met Leu
25 30 35 40

AGC CTC CCT GGC CAG TTC ACC TTC ACC GCT GAC CAG CCG CAG CTG CAC 357
Ser Leu Pro Gly Gln Phe Thr Phe Thr Ala Asp Gln Pro Gln Leu His
45 50 55

TGC GCC GCC TTC TTC ATC GGC GAG CCG GAG GAG TTC ATC ACC ATC CAC 405
Cys Ala Ala Phe Phe Ile Gly Glu Pro Glu Glu Phe Ile Thr Ile His
60 65 70

TTT GAC CTG GTC TCC ATC GAC TGC CAG GGT GGG GAT TTC CTG AAG GTA 453
Phe Asp Leu Val Ser Ile Asp Cys Gln Gly Gly Asp Phe Leu Lys Val
75 80 85

TTT GAT GGT TGG ATC CTT AAG GGG GAG AAG TTC CCA AGT TCT CAG GAT 501
Phe Asp Gly Trp Ile Leu Lys Gly Glu Lys Phe Pro Ser Ser Gln Asp
90 95 100

CAC CCT CTG CCC ACC AGG GAG AGG TAC ACA GAT TTC TGT GAG AGC GGT 549
His Pro Leu Pro Thr Arg Glu Arg Tyr Thr Asp Phe Cys Glu Ser Gly
105 110 115 120

CTC ACC AGA AGG AGT GTT ACA TCT TCC CAG AAT GTG GCC ATG GTC TTC 597
Leu Thr Arg Arg Ser Val Thr Ser Ser Gln Asn Val Ala Met Val Phe
125 130 135

TTC CGG GTC CAT GAA CCA GGA AAT GGA TTC ACG ATA ACC ATA AAG ACA 645
Phe Arg Val His Glu Pro Gly Asn Gly Phe Thr Ile Thr Ile Lys Thr
140 145 150

GAC CCC AAC CTC TTC CCT TGC AAT ATC ATC TCT CAG ACT CCG AGT GGA 693
Asp Pro Asn Leu Phe Pro Cys Asn Ile Ile Ser Gln Thr Pro Ser Gly
155 160 165

AGA TTT GCT TTG GTG GTT CCA TAC CAG CAC CAA AAC TGC AGC TTT TCC 741
Arg Phe Ala Leu Val Val Pro Tyr Gln His Gln Asn Cys Ser Phe Ser
170 175 180

ATC ATT TAT CCG GTG ACC ATC AAA ATC TCT GAC CTC GCC CTG GGA CAC 789
Ile Ile Tyr Pro Val Thr Ile Lys Ile Ser Asp Leu Ala Leu Gly His
185 190 195 200

CTG CAT GGC CTT CAG TTG AAG AAA CCT GCG GCT GGC TGT GGT GGA ACT 837
Leu His Gly Leu Gln Leu Lys Lys Pro Ala Ala Gly Cys Gly Gly Thr
205 210 215

GGA GAC TTT GTG GAG CTG CTG GGA GGA ACT GGA CTG GAC ACC TCC AAG 885
Gly Asp Phe Val Glu Leu Leu Gly Gly Thr Gly Leu Asp Thr Ser Lys
220 225 230

ATG ATG CTC TTA GTG GAC CTG TGT TAC CCC TTT CAT GGC CCT GCC CAG 933
Met Met Leu Leu Val Asp Leu Cys Tyr Pro Phe His Gly Pro Ala Gln
235 240 245

ATG AAA ATT AGC TGC GAC AAT GCT GTG GTG AGG ATG GTC TCC AGT GGA 981
Met Lys Ile Ser Cys Asp Asn Ala Val Val Arg Met Val Ser Ser Gly
250 255 260

AAA CAC ATG AAC CGT GTG ACT TTT GAG TAT CGT CAG CTG GAA CCA CTC 1029
Lys His Met Asn Arg Val Thr Phe Glu Tyr Arg Gln Leu Glu Pro Leu
265 270 275 280

GAG CTG GAA ACC TCG ACC AGA AAC AGC ATC CCG GAG TAC TGC TTG TCT 1077
Glu Leu Glu Thr Ser Thr Arg Asn Ser Ile Pro Glu Tyr Cys Leu Ser
285 290 295

AGT CTT TGA ATGACCAGC 1095
Ser Leu .